# EUROPEAN PATENT APPLICATION

(11) **EP 4 523 618 A1**
(43) Date of publication of application: **19.03.2025**
(21) Application number: 24200682.3
(22) Date of filing: 17.09.2024
(51) Int. Cl.: A61B 5/06, A61B 34/20

(54) **A SYSTEM OF PROCESSING OF LOCATION SIGNALS**

(30) Priority: 18.09.2023 US 202318369230
(71) Applicant: Biosense Webster (Israel) Ltd., 2066717 Yokneam (IL)
(72) Inventor: GOVARI, Assaf, 2066717 Yokneam (IL); GLINER, Vadim, 2066717 Yokneam (IL); ALTMANN, Andres Claudio, 2066717 Yokneam (IL)
(74) Representative: Carpmaels & Ransford LLP

(57) **Abstract**

A methods and systems are disclosed to determine positions of one or more distal end portions of a catheter. In embodiments, the method includes processing location signals that are transmitted by a transmitter-receiver utility having a first clock, and received by a position sensor at a catheter's distal-end portion. The processing may be performed by a signal processor having a second clock not synchronized with the first clock of the transmitter-receiver utility. The processing includes: determining a clock-rate-ratio between clock-rates of the first and second clocks; determining a time-lag between the first and second clocks; and determining the relative phases of the received signals while compensating for the clock-rate-ratio and for the time-lag between the clocks, such that determined phases are adjusted relative to the first signal clock of the transmitter receiver utility. Accordingly, the position of the catheter's distal-end portion can be determined based on the relative phases.

## Description

### TECHNOLOGICAL FIELD

The present invention is in the field of medical devices' signals and particularly relates to determining the positions of multiple medical instruments or other portions thereof during medical procedures.

### BACKGROUND

A wide range of medical procedures involve a plurality of medical devices on and/or within a patient's body. The medical devices may be for example one or more catheters, and in many cases a single catheter includes a plurality of portions/sections, which may be for example one or more flexible sections (such as flexible arms/splines thereof) and/or medical instruments which may be distributed on the flexible sections (e.g., sensors/probs, electrodes such as EEG electrodes, ablation instruments and/or other instruments). During a medical operation the flexible sections of such catheters may be flexed/bent to simultaneously approach/contact different parts of the treated anatomy, for sensing (mapping) and/or treating the tissue at those different parts by the medical instruments thereon, in some case simultaneously. One medical procedure in which the use of catheters including a plurality of flexible sections/portions have proved extremely useful is in the treatment of cardiac arrhythmias. Cardiac arrhythmias and atrial fibrillation in particular, persist as common and dangerous medical ailments, especially in the aging population.

Diagnosis and treatment of cardiac arrhythmias include mapping the electrical properties of heart tissue, especially the endocardium and the heart volume, and selectively ablating cardiac tissue by application of energy. Often in such procedures, one or more catheters having a plurality of portions such as flexible sections/splines, are inserted into the patient body. In some cases, medical instruments arranged on these plurality of portions are operated for carrying out multiple interrelated operations such as electrocardiogram (ECG) mapping, tissue ablation, temperature sensing/mapping and/or image/ultrasound sensing. Some catheters are dedicated for placement in specific parts of the anatomy, e.g., heart chamber, coronary sinus, esophagus, atrium, ventricle, and include multiple flexible sections/portions that can conform to the shape of the anatomy of the treated body part, and optionally having a plurality instrument arranged at the different portions.

### BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:
**Fig. 1** is a schematic illustration showing a catheter-based mapping and ablation system according to an embodiment of the present invention;
**Fig. 2** is a schematic illustration of a catheter having multiple portions / medical instruments at a distal end thereof and position sensors associated therewith;
**Fig. 3A** is a block diagram illustrating the configuration of a positioning system **100** according to an embodiment of the present invention;
**Fig. 3B** is a flow diagram illustrating a method **200** to determine positions of medical devices and/or of distal end portions thereof according to embodiments of the present invention;

In some figures, like reference numerals are used to designate similar modules/elements of the present invention or elements/modules having like functionalities. Accordingly, unless otherwise specified, description of modules/elements with reference to a certain embodiment of the invention should be understood to apply to all embodiments of the present invention in which such module/element is incorporated.

### DETAILED DESCRIPTION OF EMBODIMENTS

With advancements of medical technologies, the number of medical devices and medical instruments that are involved in a medical procedure and spatially arranged, placed or moved, within the patient's body has increased significantly. For example, in some medical procedures, catheters, such as intracardiac ECG mapping catheters and/or ablation catheters, having a plurality of distal end portions, such as movable/flexible sections (e.g., splines) and/or medical instruments are used. For example, in many cases the medical instruments are arranged at various movable/flexible sections of the catheter's distal tip, such as the shaft, distal end effector, splines and/or at various other sections of the distal end effector thereof. The movable/flexible sections of the catheter may be adapted for flexing/expanding during the medical procedure (e.g., in order to accommodate the volume of the treated/monitored body anatomy and/or for bringing medical instruments thereon into contact or close proximity, with the wall/boundary of the treated/monitored body anatomy. Such techniques are used for example in order to enable mapping and/or treating several tissue regions of the anatomy substantially simultaneously. To this end, a large number of medical instruments, such as ECG electrodes, ablation utilities/instruments or other instruments, which may be arranged at a plurality of movable/flexible sections of a catheter, and may for example be used to enable a physician to simultaneously capture/map the electrical activity from multiple locations in the heart chamber, and/or or to simultaneously ablate specific tissue regions thereof.

In order to achieve such medical procedures with accuracy, the positions of the medical instruments furnished at the different flexible/movable sections of the catheter, and/or the positions of one or more of the flexible/movable sections of the catheter themselves should be monitored/tracked relative to treated/probed anatomy of the patient's body.

For clarity, in the following, the phrase *distal end portions* of a medical device/catheter, and variants thereof is used to designate portions/elements/sections that are arranged at a distal tip/end of a medical device/catheter, and whose positions should be determined during a medical procedure. In this regard it should be understood that the phrase *distal end portions* may encompasses any one of flexible/movable section(s) of a catheter's distal tip, and/or medical instruments arranged thereon, which are associated with position sensors located adjacent thereto and capable of providing data/signals indicative of the respective positions of the *distal end portions.* The term *position* is used herein to designate any one of a location, orientation or both, of a medical device or of a distal end portion thereof. For instance, in some case there may not be a need to determine the location of a medical-device or of portion thereof, but only its orientation, or vice-*versa,* and the system of the present invention may be adapted/operable to achieve the same.

To achieve that medical device/catheter may be configured such that different distal end portions thereof, or some of them, include respective position sensors capable of receiving location signals (e.g., electromagnetic signals) by which the positions of the different portions can be determined. These received location signals should then processed in order to monitor/track the positions of different portions of the catheter's distal end a during the medical procedure. Accordingly in this way valuable information about the positions of different portions of the medical device/catheter can be provided to the physician during the medical procedure (e.g., in real-time).

In view of the above there is a need in the art for systems and methods to enable determining the positions of the plurality of portions of a distal end of a catheter or other medical devices having different portions thereof movable with respect to one another and/or with respect to a main body/shaft of the device. Conventional hardware for determining the positions of medical devices, are often limited in their capacity to determine a plurality of discrete positions from pluralities of position sensors such as those furnished on different distal end portions of catheters of the type described above (as various catheters of this type may include between few and few tens of position sensors). To this end, the conventional hardware of medical device position determination is generally not suited for connecting to medical devices such as catheters, having relatively large pluralities of position sensors whose positions need to be determined. Indeed, the conventional hardware typically includes transmitters for transmitting the location signals that can be received by the plurality position sensors of such catheters or other medical devices, but has limited channels for collecting the locations signals received by the plurality of position sensors of such devices, and cannot accommodate simultaneous processing of location signals provided from such pluralities of positions sensors that are embedded in some of the advanced medical devices, such as spline or basket type catheters which have a plurality of respectively movable portions at their distal end/tip.

The present invention therefore provides novel systems and methods for solving these problems to enable extending the capabilities of existing medical device positioning hardware to enable determining (e.g., simultaneously), the positions of multiple portions of medical devices, while utilizing, and in synchronization with, location signals that are provided/transmitted by the existing conventional medical device positioning hardware. Advantageously the invention facilitates the positioning of relatively large pluralities of position sensors which may not be facilitated by the conventional positioning hardware, while without a need to replace the existing positioning hardware (e.g., which may already be deployed) and without addition of location signal transmitters or transmission of additional location signal. Accordingly, the positions of large number of medical devices/instruments or portions thereof may be determined during a medical procedure in cost effective manner, and without overcrowding the medical facilities with additional electromagnetic signals.

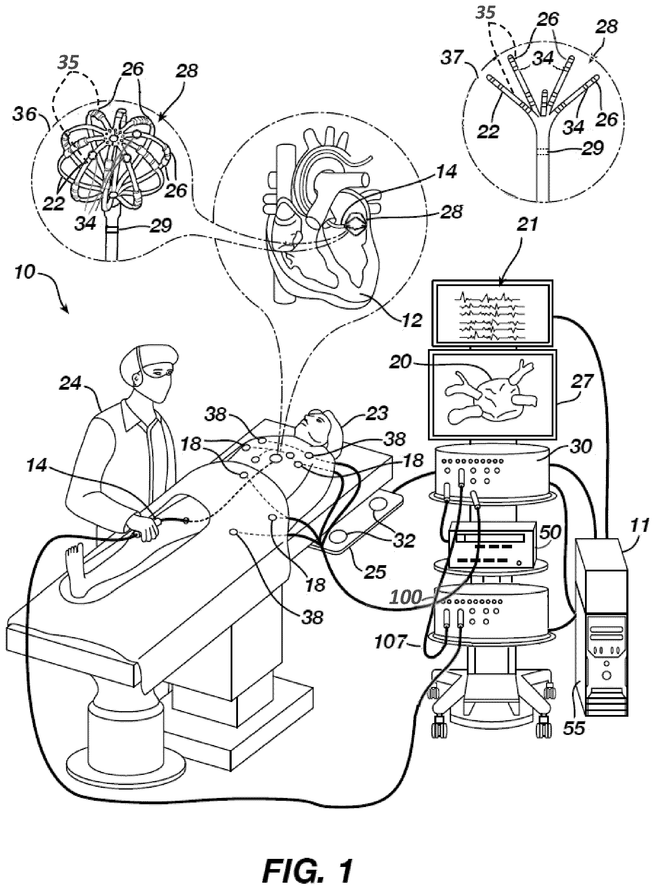

Reference is made to **Fig. 1** showing an example catheter-based mapping and ablation system **10.** System **10** includes or is connectable to multiple medical devices **14 and** may include for example catheters **36** and **37**, whose positions, and preferably the positions of the various distal end portions **35** thereof, should be determined by the system **10.** It should be noted that the distal end portions **35** whose positions are to be determined, a may include the main body (e.g. a catheter's shaft) **14H** of a medical device **14** such as catheter **36** or **37**, and/or one or more flexible/movable sections/splines **22** thereof, and/or medical instruments **26** furnished at the distal end of the medical device **14** (e.g. being movable relative to the main body/shaft of the medical device **14**) and whose positions therefore may not be accurately determined based only on measurements of the position of the medical device itself. In the none-limiting examples provided herein the distal end portions **35** include some of the medical instruments **26** and/or the movable sections/splines **22** at which they are furnished in the catheters **36** and/or **37**. It should also be noted that the movable sections **22** may be implemented using various configurations such as flexible splines as shown in the figure, or by other configurations for example utilizing pivots, flexible joints, extendable/telescopic shafts or any other configuration without departing from the scope of the present invention.

Catheters **36** and/or **37** may be percutaneously inserted by a physician **24** through the patient's vascular system into a chamber or vascular structure of a heart **12**. Typically, a delivery sheath catheter is inserted into the left or right atrium near a desired location in heart **12**. Thereafter, one or more catheters, such as **36** and/or **37**, may be inserted into the delivery sheath catheter so as to arrive at the desired location in heart **12**. For example, the system **10** may include catheters dedicated for sensing Intracardiac Electrogram (IEGM) signals, such as catheter **37**, catheters dedicated for ablating, such as catheter **36**, catheters dedicated for both sensing and ablating (not specifically shown in the figure), and/or other medical devices. Examples for catheters **37** and **36**, which are configured for sensing IEGM and for ablating are respectively illustrated herein. Physician **24** may place a distal tip **28** of any of the catheters **14** such that multiple medical instruments **26** thereof, which are arranged on the flexible/movable sections **22** of the catheter **14** can move/flex to approach/contact the heart wall for probing/treating target sites in heart **12.** For sensing IEGM signals and/or for ablating a tissue, physician **24** may place a distal end **28** of the respective sensing or ablation catheter, **37** or **36**, such that the medical instruments **26** thereof approach/contact with tissue at the target site.

Catheters **36** and **37** shown in the figure exemplify medical devices **14** whose positions, and/or the positions of at least some distal end portions **35** thereof (e.g., medical instruments **26** and/or movable sections **22**) should be determined (tracked/monitored) by the system **10**. Magnetic based position sensors **29** and/or **34** located on the catheter's distal tip **28** may be operated to provide data indicative of the real-time positions of the distal end portions **35** of the catheters during the medical procedure. The magnetic based position sensors **29** and **34** operate in conjunction with location pad **25** which includes a plurality of location signal transmitters **32** (e.g., magnetic coils) that generate/transmit electro-magnetic location signals (e.g., magnetic fields) in a predefined working volume surrounding the patient. Real time position of the distal tip **28** of the catheter **36** / **37** and/or of the distal end portions **35** thereof may then be tracked based on the electro-magnetic location signals that are generated with location pad **25** and sensed by magnetic based position sensor **29** and/or **34.** Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

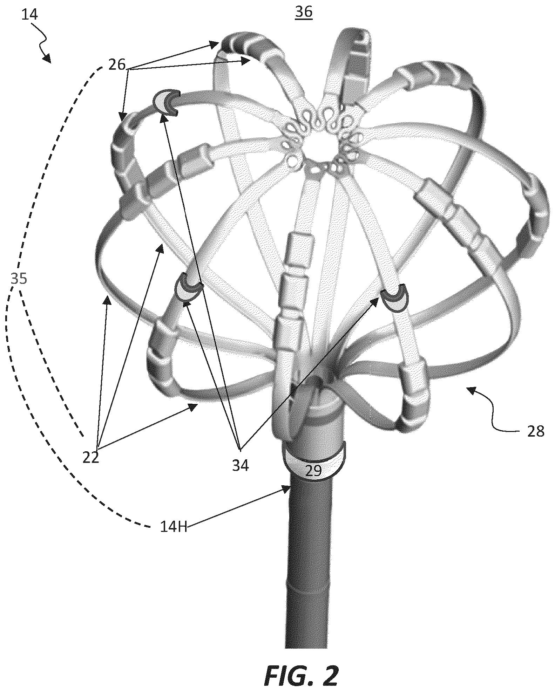

Catheters **36** and **37** shown in **Fig. 1**, exemplify medical devices **14** that are connectable to the system **10** and each includes multiple distal end portions **35** including in these examples flexible sections **22** (splines or basket arms) extending from the distal tips **28** of the catheters **36** and **37** and medical instruments **26** arranged thereon. With reference to **Fig. 2**, a perspective view of an ablation catheter **36** manifesting an example of a medical device **14** with movable distal end portions **35** is illustrated. The catheter **36** includes a shaft **14H**having a distal end/tip **28.** The catheter **36** in this non-limiting example includes a plurality of distal end portions **35** arranged at the distal end/tip **28** including flexible sections **22** (hereinafter also referred to as *splines*) and multiple medical instruments **26** arranged thereon. The splines **22** arranged near/at the distal end **28** form and expandable distal end assembly that can be deflectable outwardly from the catheter by moving the pusher rode **14P** to thereby engage the medical instruments **26** to approach/contact the tissue wall of the heart for ablation of desired regions of the tissue. In some embodiments, such as in catheter **37**, medical instruments **26** may include for example near-field intracardial electrogram sensors/electrodes for sensing near-field signals IEGM signals (e.g., activation biopotential signals) when the catheter is located intracardially and the instruments **26** are approached to the tissue. In the non-limiting example of **Fig. 2**, the flexible sections **22** are connected to one another in a "basket" like form. In other examples, the flexible sections **22** of the catheter may be connected to one another in a different configuration and/or may be independently movable such as in catheter **37** shown in **Fig.1**, and/or may have different shapes/configurations (e.g., other than splines).

Thus, the system of the present invention may be configured and operable for determining the position of a medical device(s) **14** connected thereto; e.g., catheter(s) **36** and/or **37**, and/or of distal end portions **35** thereof. The catheter, for example **36,** may include a plurality of position sensors e.g., **29** and **34** enabling the system the system **10** of the present invention to determine the positions of distal end portions **35.** In this none-limiting example catheter **36** includes a position sensor **29** embedded in the main body of the catheter (e.g., in or near the distal tip **28** thereof) to enable tracking the location and/or orientation of distal tip **28** of the catheter **36.** Additionally, in this none-limiting example catheter **36** includes a plurality of position sensors **34** arranged at the movable sections **22** or other distal end portions **35** thereof and, whose positions can also be tracked by the system **10.** The position sensors **29** and/or **34** are adapted to receive location signals that are transmitted by a location signal transmitter receiver utility of the system **10**, and to provide the received signals for processing by system **10.** The location signals obtained from the position sensors **29** and/or **34**, enable the system **10** to determine the respective positions of some or all of the distal end portions **35** of the catheter **36**, such as the positions of the flexible sections **22** thereof and/or the positions of medical instruments thereon **26**, as well as typically the position of the main-body/distal-tip **28** of the catheter **36.** In some embodiments the system **10** may utilize the signals from the position sensors **34** to determine/assess the shape(s) of the distal end flexible sections **22** of the catheter while they being flexed/shifted during the medical procedure.

Typically, the position sensors **29** and/or **34** are magnetic/electromagnetic based position sensors. In some embodiments, the position sensors such as **29** may be implemented as a three axes sensor (TAS) including three magnetic coils to enable sensing of three-dimensional (3D) location and orientation thereof. In some embodiments, the position sensors, such as **34,** may be implemented as single-axis sensor (SAS) including one magnetic coil to enable axial sensing of their orientation (e.g., whereby the axial position sensing obtained by SAS sensors may be for instance combined with three-dimensional (3D) position and orientation sensing of another position sensor of the catheter, such as **29**, to facilitate determining the locations of the SAS sensors). It should be noted that the catheter **37** illustrated in **Fig. 1** may in some cases have similar configuration as catheter **36** describe above, although the flexible sections **22** may be configured differently, e.g., not necessarily connected/constrained to one another in basket form.

Turning back to **Fig. 1****,** system **10** may also include one or more electrode patches **38** positioned for skin contact on patient **23** to establish location reference for location pad **25.** Optionally in some embodiments one or more of the distal end portions **35** include ECG sensors/electrodes, and the location pad **25** may also be used for locating such distal end portions by impedance-based location tracking. Impedance-based location tracking may be used for example for locating distal end portions **35** that are not associated/fumished with position sensors capable of sensing their accurate positions, provided that the distal end portions **35** are furnished with electrodes suitable for determining their positions utilizing impedance-based tracking techniques. For impedance-based tracking, electrical current is directed to electrodes (e.g., ECG electrodes in case such are included at/on the distal end portion 35). The electrical current is then sensed at electrode skin patches **38** so that the location of each ECG electrode can be triangulated via the electrode patches **38.** Details of the impedance-based location tracking technology are described in US Patent Nos. 7,536,218; 7,756,576; 7,848,787; 7,869,865; and 8,456,182.

System **10** may also include a recorder **11** records and displays electrograms **21** captured with body surface electrocardiogram (ECG) electrodes **18** and intracardiac electrograms (IEGM) captured with ECG electrodes of one of the medical devices/catheters **14** which may be connected to the system. Recorder **11** may include pacing capability for pacing the heart rhythm and/or may be electrically connected to a standalone pacer.

System **10** may include an ablation energy generator **50** that is adapted to conduct ablative energy to one or more of electrodes at a distal tip of one of the catheters **14** that configured for ablating. Energy produced by ablation energy generator **50** may include, but is not limited to, radiofrequency (RF) energy or pulsed-field ablation (PFA) energy, including monopolar or bipolar high-voltage DC pulses as may be used to effect irreversible electroporation (IRE), or combinations thereof.

System **10** typically include a Patient interface unit (PIU) **30**, which is an interface configured to establish electrical communication between medical devices, such as catheters and/or other electrophysiological equipment, and a workstation **55** for controlling operation of system **10**. The medical devices of system **10** include may include for example electrophysiological equipment such as one or more catheters **14**, location pad **25**, body surface ECG electrodes **18**, electrode patches **38**, ablation energy generator **50**, and recorder **11**. Optionally and preferably, PIU **30** additionally includes processing capability for performing ECG calculations and/or for implementing real-time computations of location of some of the medical devices/catheters connected to the system.

Workstation **55** includes memory, processor unit with memory or storage with appropriate operating software stored therein, and user interface capability. Workstation **55** may provide multiple functions, optionally including (1) modeling the endocardial anatomy in three-dimensions (3D) and rendering the model or anatomical map **20** for display on a display device **27**, (2) displaying on display device **27** activation sequences (or other data) compiled from recorded electrograms **21** in representative visual indicia or imagery superimposed on the rendered anatomical map, (3) displaying real-time location and orientation of multiple catheters within the heart chamber, and (4) displaying on display device **27** sites of interest such as places where ablation energy has been applied. One commercial product embodying elements of the system **10** is available as the CARTO^{™} 3 System, available from Biosense Webster, Inc., 31A Technology Drive, Irvine, CA 92618.

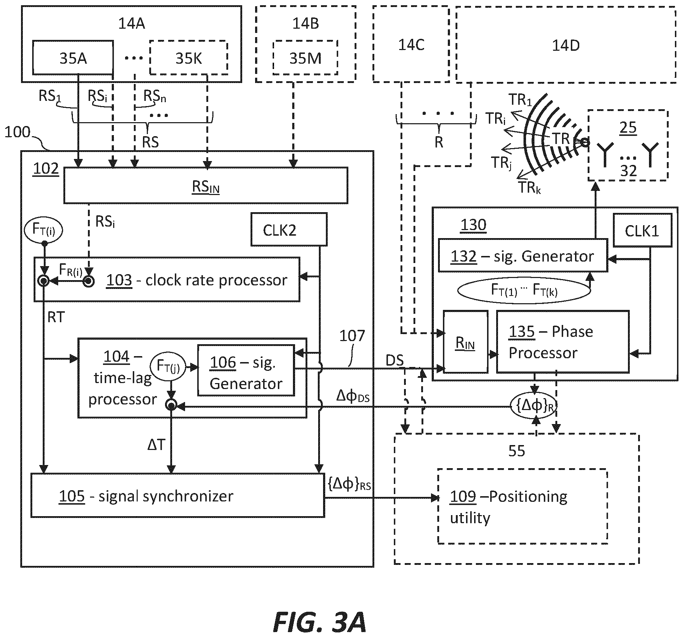

As indicated above, typically, the PIU **30** typically implements processing capability for implementing real-time computations of positions of some of the medical devices/catheters **14** that are connected to the system **10.** To achieve that the PIU **30** typically includes a transmitter-receiver utility (not specifically shown in **Fig. 1**; **130** in **Fig. 3A**) capable of transmitting location signals and obtained the received signals which are received in responsive to said transmissions by positions sensors associated with the medical modules, and applying real-time processing to the received signals to determine data indicative of the positions of at least some of the medical devices **14**, which are connected to the system **10.** Specifically, the transmitter-receiver utility of the PIU **30** includes a signal generator that is connected to the location pad **25** and adapted to generate electromagnetic location signals of certain plurality of frequencies. The location signals are then transmitted by the location pad **25** and received by the position sensors **29** and/or **34** of the medical devices/modules **14**/**26** that are connected to the system **10.**

In turn the transmitter-receiver utility of the PIU **30** is connected to some of the medical devices **14** and is adapted to collect and process the location signals that were received by their position sensors and determine phases relative to the transmitted location signals, based on which the positions of the medical modules/devices can be determined. The transmitter-receiver utility of the PIU **30** may be for example adapted to apply analog to digital conversion (sampling) to the position signals received from the position sensors of the medical devices that are connected thereto, to determine their phases relative to the location signal transmissions made thereby, and provide/communicate the same, (or the actual positions calculated from the phases) to the workstation **55**, for further processing or display to the physician.

However, in some implementations of systems **10** (e.g., systems already deployed in medical operation facilities), the PIU **30** may have limited input channels for collecting and/or simultaneously sampling location signals and is not adapted to accommodate sufficient number of input channels for the multitude of position sensors which are included implemented in advanced medical devices **14** having a plurality of distal end portions **35** whose positions should be separately determined.

Therefore, in some implementation system **10** includes an additional positioning system **100** which is configured and operable for complementing/extending the functionality of the PIU **30** to enable connecting to the system **10**, additional channels for position sensors of additional medical devices, such as catheters **36** or **37**, which have one or more respectively movable distal end portions **35** at the distal end **28** thereof.

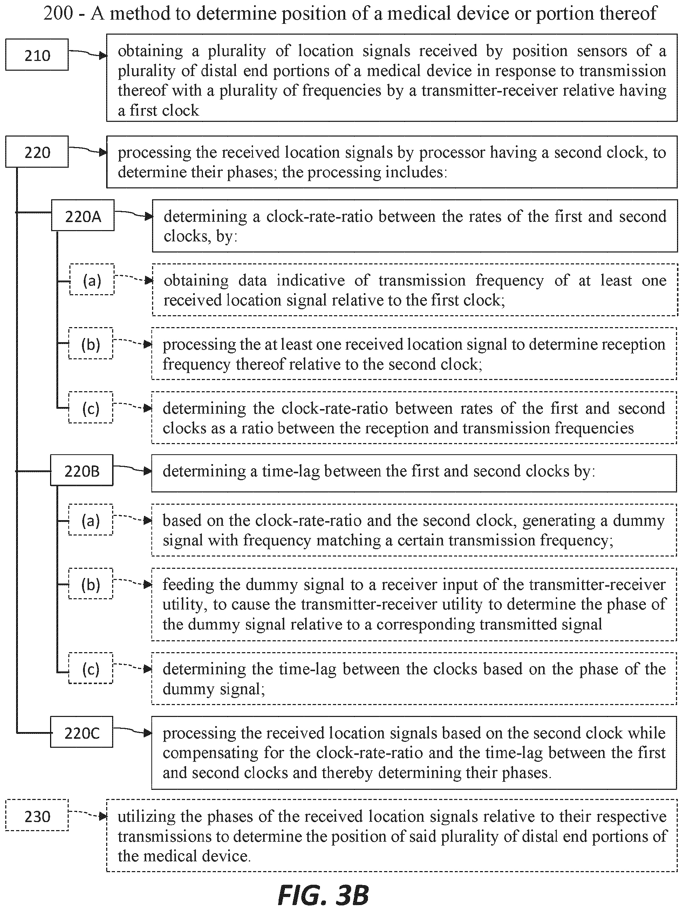

Reference is now made together to **Figs. 3A** and **3B** in which a block diagram of a positioning system **100** and a flow chart of a method **200** according to embodiments of the present invention are respectively illustrated. **Fig. 3A** depicts the configuration of the system **100**, which is adapted to determine positions of a plurality of distal end portions **35A-35K** and **35M** of one or more medical devices e.g. **14A** and **14B.** **Fig. 3B** illustrated the method **200** for determining the positions of distal end portions such as **35A-35K** and **35M.** Method **200** is implemented by the system **100** and accordingly, for brevity embodiments of the system **100** and the method **200** of the present invention are described herein below together with reference to the **Figs. 3A** and **3B****.**

The system **100** includes a signal processor **102** that is configured and operable for connection with the position sensors (e.g. **29** and/or **34**) in **Fig. 1** associated with one or more distal end portions, such **35A** to **35K, 35M** of medical devices **14A** and/or **14B** and/or other medical devices or distal end portions thereof, to receive therefrom a plurality of location signals **RS** (hereinafter also refer to as *received signals*) received by position sensors **29** / **34** associated therewith, and determine the phases of the received location signals **RS,** by which the positions of the distal end portions or some of them can be inferred.

In the present non-limiting example, medical modules **35A** to **35K, 35M** are shown to be associated-with/parts of medical devices **14A** and **14B** respectively. The position sensors of the medical devices **14A** and **14B** and/or of the distal end portions thereof, **35A** to **35K** and **35M**, are connected to signal processor **102** for inference of data indicative of their respective positions, by the signal processor **102.** It should be understood that the present invention may be implemented with a different number of medical devices and associated distal end portions **35** that can be connected to the signal processor **102**, and the specific number of medical devices and distal end portions shown in the figure are provided herein as an example and should not be limiting.

In **Fig. 3A****,** additional medical devices **14C** and **14D** which may each have one or more position sensors at the distal tip thereof are illustrated as connected to a location transmitter receiver utility **130** (e.g., of PIU **30** shown in **Fig. 1**). Typically, the location transmitter-receiver utility **130**, which may or may not be a part of the system **100**, includes a signal generator **132** that is connectable to a plurality of location signal transmitters **32**, which are typically arranged in location pad **25** (e.g., the location pad **25** is intended for placement near/underneath a patient being treated to enable locating the medical devices used in the medical procedure). The signal generator **132** of the transmitter-receiver utility **130** is configured and operable to generate a plurality of location transmission signals **TR₁** to **TRₖ** (hereinafter *transmissions* or *transmitted signals*) having a plurality of respective transmission frequencies **F_{T(1)}** to **F_{T(k)}** (e.g., being predetermined frequencies). The signal generator **132** provides the transmission signals **TR₁** to **TRₖ** to the location signal transmitters **32**, by which they are transmitted as wireless electromagnetic signals (e.g., magnetic fields). In some embodiments the transmission signals **TR₁** to **TRₖ** are continuous-wave (CW) signals having the different frequencies **F_{T(1)}** to **F_{T(k)}**.

In turn, one or more of the transmitted signals **TR₁** to **TRₖ** are received/sensed by the position sensors of the medical devices **14A** to **14D** whose positions of the positions of one or more distal end portions thereof are to be determined. The transmitter-receiver utility **130** typically includes receiver input ports/channels **R_{IN}** and a phase processor **135** by which it can be connected to one or more (typically a plurality) of the medical devices, such as to medical devices **14C** and **14D** (i.e., to the position sensors associated therewith), to obtain the locations signals **R** received thereby. The received location signals **R** from medical devices **14C** and **14D** are processed by the transmitter-receiver utility **130** to determine their respective phases **{Δφ}_{R}** relative to the corresponding transmitted signals **TR₁** to **TRₖ**. The processing in this case, is made by transmitter-receiver utility **130** and therefore relies on the same clock **CLK1** of the transmitter-receiver utility **130** which is used for the generation of the transmitted signals **TR₁** to **TRₖ.** Accordingly, determining the relative phases of the location signals **R** received by the position sensors of the medical devices **14C** and **14D** that are connected transmitter-receiver utility **130** can be done accurately in straight forward manner by using the same clock which is used for the signals' transmissions. As would be readily appreciated by those versed in the art, in this manner the positions of these medical devices **14C** and **14D** can be inferred based on the relative phases of the received location signals **R** that are determined by the transmitter-receiver utility **130**.

However, as indicated above, the transmitter-receiver utility **130** has a limited number of input ports/channels **R_{IN}** and can obtain and process only a limited number of received location signals **R** from a limited number of position sensors of the medical devices such as **14C** and **14D.** With the advent of medical technologies and the increase in numbers of position sensors such as **29** and **34** furnished at pluralities of distal end portions of medical devices, such as **14A** and **14B,** there is a need to supplement the signal processing capacity of the transmitter-receiver utility **130** to facilitate inference of the positions of the pluralities of distal end portions **35A-35K** and **35M** of such devices (since their number is often in excess of the number of input channels **R_{IN}** of the transmitter-receiver utility **130**).

In order to achieve that, the system **100** includes the signal processor **102** indicated above. The signal processor **102** has additional input channels/ports **RS_{IN}** for receiving additional location signals **RS** from the positions sensors **29/34** that are associated with the distal end portions e.g., **35A-35K** and **35M** of one or more of the medical devices **14A** and/or **14B** that are connected to the system **100.** In this regard it should be noted that hereinafter the phrases *received signals* and/or *location signals* designated by **RS** are used to refer to the location signals received from the position sensors furnished at medical devices such as **14A** and/or **14B** and/or on the distal end portions thereof **35A** to **35K** and **35M**, which are connected to the signal processor **102** (in other words the location signals **RS** should be distinguished from the location signals **R** of the medical devices such as **14C** and **14D** which are processed by the transmitter-receiver utility **130**).Having said that, it should also be noted that the transmitted signals **TR₁** to **TRₖ** that are used for the positioning of the medical devices **14A and 14B** (e.g. and/or the distal portions **35A** to **35K** and **35M** thereof) whose position sensors are connected to the signal processor **102,** are typically the same signals **TR₁** to **TRₖ** that are transmitted by transmitter-receiver utility **130** and used for the positioning of the medical devices **14C** and **14D** whose positions sensors are connected to the transmitter-receiver utility **130.** This is preferable in various implementations of the system for example for reasons of cost effectiveness as well as in order not to over crowd the medical operation facility with additional electromagnetic signals and additional equipment for their generation.

The signal processor **102** is thus configured and operable to process the location signals **RS** received/sensed by the position sensors of the medical devices **14A** and **14B** to infer data indicative of their positions and/or of the positions of the distal end portions thereof. To this end, in line with operations **210** of method **200,** the signal processor **102** obtains the received location signals **RS** from the position sensors associated with distal end portions **35A-35K** and **35M** of the medical devices **14A** and **14B** connected thereto, for example via the input channels/ports **RS_{IN}** of the signal processor **102.** The signal processor **102** then implements the operation **220** of method **200** and processes the location signals **RS** to determine their respective phases **{φ}_{RS}** relative to corresponding ones of the transmitted signals **TR₁** to **TRₖ** which have the same frequencies.

As indicated above, the frequencies of the transmitted signals **TR** are typically set by the transmitter-receiver utility **130** based on the first clock **CLK1** being the clock of the transmitter-receiver utility **130,** while the processing of the received location signals **RS** is in this case performed by the signal processor **102** based on a second clock **CLK2** being the clock of the signal processor **102.** The first and second clocks, **CLK1** and **CLK2,** are not necessarily synchronized and may generally operate with somewhat different respective first and second clock rates and/or have time-lag between them. Therefore, a naive attempt to infer the frequencies of the received signals **RS** based on the clock **CLK2** of the signal processor **102** (and thereby also infer their phases), would generally result with a discrepancy between the frequencies of the transmitted signals **TR** and the inferred frequencies of the corresponding received signals **RS.** This may in turn yield erroneous inferred relative phases between them (e.g., as inference of the phase depends on frequency as well as the time-lag /difference between the clocks).

The signal processor **102** is therefore configured and operable mitigate such discrepancies of frequencies and/or time lag between the clocks, which might be caused by the lack of clock synchronization. In some embodiments the signal processor **102** is configured and operable as follows according to operation **220** of method **200** in order to determine the phases **{φ_{RS}}** of the received signals **RS** relative to their respective transmissions **TR₁** to **TRₖ**, with accuracy. The signal processor **102** includes: a clock rate processor **103** that is adapted to implement the operation **220A** of method **200** and determine the clock-rate-ratio **RT** between the first rate of the first clock **CLK1** of the transmitter-receiver utility **130** and the second rate of the second clock **CLK2** of the signal processor **102;** a time lag processor **104** that is adapted to implement the operation **220B** of method **200** to determine the time-lag **ΔT** between the first and second signal clocks, **CLK1** and **CLK2,** of the transmitter-receiver utility **130** and the signal processor **102** respectively; and a signal synchronizer **105** that is adapted to implement the operation **220C** of method **200** and digitize the received signals **RS** with compensation for the clock-rate-ratio **RT** and the time-lag **ΔT** determined by the clock rate processor **103** and the time lag processor **104** respectively.

More specifically, in some embodiments the clock rate processor **103** is configured and operable to determine the clock-rate-ratio **RT** between the first and second clock-rates, by carrying out the following:
(a) obtain data indicative of a transmission frequency **F_{T(i)}** of at least one received location signal e.g., **RSᵢ**, of the location signals **RS,** relative to the first clock-rate of the first clock **CLK1** of the transmitter-receiver utility **130** (i.e., obtain data indicative of the frequency **F_{T(i)}** by which the transmitted signal **TRᵢ** which corresponds to the at least one received signal **RSᵢ**, was transmitted by the transmitter-receiver utility **130**). This generally implements the operation **220A** - **(a)** of method **200.**
(b) process the at least one received location signal **RSᵢ** to determine a reception frequency **F_{R(i)}** thereof relative to the second clock-rate of the second clock **CLK2** of the signal processor **102** - thus generally implementing the operation **220A** - **(b)** of method **200;** and
(c) determine the clock-rate-ratio **RT** based on the transmission and reception frequencies, **F_{T(i)}** and **F_{R(i)}**, that are inferred for the at least one location signal **RSᵢ** in (a) and (b) respectively. This generally implements the operation **220A** - **(c)** of method **200.**

The operation **220A** and its optional sub operations implemented by the clock rate processor **103** may be carried out for example based on the following. As for the transmission frequency **F_{T(i)}** obtained in **(a)**, in typical embodiments where the transmitter-receiver utility **130** utilizes predetermined/preset transmission frequencies **F_{T(1)}** to **F_{T(k)}** for the transmitted signals, the clock rate processor **103** may be preconfigured or adjusted to operate according to a certain one **F_{T(i)}** (or more) of these predetermined/preset transmission frequencies. Alternatively, in cases where the transmission frequencies **F_{T(1)}** to **F_{T(k)}** are not a-priori fixed, the clock rate processor **103** may be adapted to obtain data indicative of one or more of them from an external utility such as the transmitter-receiver utility **130** itself or from another utility, such as workstation **55**, which may hold information about the transmission frequencies by which the transmitter-receiver utility **130** operates.

As for the reception frequency **F_{R(i)}**, inferred in **(b)**, different techniques for inferring this frequency **F_{R(i)}** from the received signal **RSᵢ** may be implemented in various embodiments of the invention. For instance, in some embodiments the clock rate processor **103** digitizes the received signal **RSᵢ** (e.g., samples it) based on the clock rate of the second clock **CLK2** and thereby yields a digitized received signal. Then the clock rate processor **103** applies *tone detection processing* (e.g., spectral analysis), such as Fourier-Transform (e.g., FFT), to the digitized signal, and thereby determines the frequency **F_{R(i)}** of the received signal **RSᵢ** relative to the second clock **CLK2.** As would be appreciated by those versed in the art, in this manner the frequency of the received signal **RSᵢ** relative to the second clock can be determined by identification of a prominent peak in the Fourier-Transform. Alternatively, or additionally, in some embodiments the clock rate processor **103** include a Clock-Derivation processor (not specifically illustrated in the figure; typically comprising a comparator and a counter) that is connected to the second clock **CLK2** and to the input channels/ports **RS_{IN}** by which the received signal **RSᵢ** is obtained. Typically, the Clock-Derivation processor is adapted to determine a tick-count of the second clock **CLK2** between zero-crossings of the received signal **RSᵢ** and thereby infer the frequency **F_{R(i)}** of the received signal **RSᵢ** relative to second clock **CLK2** rate (in such implementations where the frequency is determined by Clock-Derivation, digitization and/or Fourier transform of the signal which may be computationally intensive, may be obviated).

Finally, the clock-rate-ratio **RT** between the first clock of transmitter-receiver utility **130** and the second clock **CLK2** of the signal processor **102** may be for example determined in **(c)** according to the ratio between the frequency **F_{R(i)}** of the received signal **RSᵢ** as determined in **(b)** and the corresponding transmission frequency **F_{T(i)}** determined in **(a).** As both these frequencies pertain to the same underlying signal, but measured by the different respective second and first clocks, **CLK2** and **CLK1,** their ratio is indicative of the ratio **RT** between the clock respective rates.

After the clock-rate-ratio **RT** is determined by the clock rate processor **103,** the operation **220B** and optionally its sub operations, is implemented by the clock rate processor time lag processor **104** as described in the following. The time lag processor **104** operates to determine the time-lag **ΔT** between the clock **CLK1** of the transmitter-receiver utility **130** and the clock **CLK2** of the signal processor **102.** To achieve that in some embodiments the time-lag processor **104** is configured and operable to carry out the following optional operations:
(a) Obtains the clock-rate-ratio **RT** determined by the clock rate processor **103,** and based on the clock-rate-ratio **RT,** generates a dummy signal **DS** with frequency that matches a certain one of the transmission frequencies of the signals **TR₁** to **TRₖ** transmitted by the transmitter-receiver utility **130.** This generally implements the operation **220B** - **(a)** of method **200.** In this non-limiting example, the dummy signal **DS** is generated with frequency **F_{T(j)}** that matches the frequency of the transmitted signal **TRⱼ**.
(b) The time lag processor **104,** then feeds the dummy signal **DS** to the receiver input **Rin** of the transmitter-receiver utility **130.** As indicated above this generally causes the transmitter-receiver utility **130** to determine the phase difference **Δφ_{DS}** between the dummy signal **DS** and the corresponding transmitted signal **TRⱼ** that is transmitted thereby with the same frequency **F_{T(j)}**. This generally implements the operation **220B - (b)** of method **200.**
(c) Subsequent to the feeding of the dummy signal **DS,** the time lag processor **104** implements operation **220B** - **(c)** of method **200.** The time lag processor **104** obtains, directly or indirectly (e.g. via workstation **55**), the phase difference **Δφ_{DS}** that is determined by the transmitter-receiver utility **130** between the dummy signal **DS** and the corresponding transmitted signal **TRⱼ**, and utilizes the frequency value **F_{T(j)}** of dummy signal **DS** and the phase difference **Δφ_{DS}** determined by transmitter-receiver utility **130** to infer the time-lag **ΔT** between the clock **CLK1** of the transmitter-receiver utility **130** and the second clock **CLK2** of the signal processor **102.** The time-lag **ΔT** between the clocks **CLK1** and **CLK2** may for example be computed as: **ΔT = Δφ_{DS} / 2πF_{T(j)}**.

More specifically in some embodiments the operation of the time lag processor **104** and operation **220B** of method **200** may be carried out for example as follows. The time lag processor **104** may include a signal generator **106** that is connected to the clock **CLK2** of the signal processor **102** (i.e., to the second clock). In **220B-(a)** the time lag processor **104** may operate the signal generator **106** to generate the dummy signal **DS** with the desired transmission frequency **F_{T(j)}**. In this regard it should be understood that as the frequency of the dummy signal should match one of the transmission frequencies of the transmitter receiver utility **130**, e.g., match the frequency **F_{T(j)}**. Therefore, as this frequency **F_{T(j)}** is set relative to the clock **CLK1** of the transmitter receiver utility **130,** in order to generate the dummy signal **DS** with that frequency based on the signal processor's second clock **CLK2,** the time lag processor **104** implements a clock-rate compensation to compensate for the difference between the clock-rate of the second clock **CLK2** that is used for the dummy signal's **DS** generation, and that of the first clock of **CLK1** of the transmitter receiver utility **130.** The clock-rate compensation is based on the clock-rate-ratio **RT** between the first and second clocks **CLK1** and CLK2 as determined by the clock rate processor **103,** and in various embodiments of the present invention may be implemented for example by any of the following techniques or by combination thereof (i) by adjusting the rate of the second clock **CLK2** by a factor of said clock rate ratio **RS** such that it matches the rate of the first clock **CLK1;** and/or or (ii) by utilizing the second clock **CLK2** (e.g. without adjusting its rate) but applying the compensation by generating the dummy signal **DS** with frequency **F_{T(j)}** multiplied by the clock rate ratio **RS.** Accordingly, the dummy signal is generated with the frequency **F_{T(j)}** set relative to the first clock **CLK1** of the transmitter receiver utility **130** such that when it is processed by the transmitter receiver utility **130** based on the first clock **CLK1,** the transmitter receiver utility **130** would "identify" it as having frequency **F_{T(j)}** matching one of its transmitted signals and would determine its relative phase accordingly.

In some implementations the system **100** includes a signal channel **107** (e.g., signal cable/communication-path) of substantially fixed and predetermined latency *Δt,* adapted for interconnecting the time lag processor **104** directly or indirectly (e.g., via workstation **55**) to an input channel **R_{IN}** of the transmitter receiver utility **130.** In such embodiments the time lag processor **104** is adapted to feed the dummy signal **DS** as input to the transmitter receiver utility **130** via that signal channel **107** having the substantially fixed latency. Then, upon receiving and processing the phase difference **Δφ_{DS}** of the dummy signal **DS** to determine the time-lag **ΔT,** the time lag processor **104** accounts for the fixed predetermined latency *Δt* of the signal channel **107** (e.g., and subtracts the latency from the computed time-lag **ΔT** to thereby obtain **ΔT** more accurately as: **ΔT** = **Δφ_{DS}** / **2πF_{T(j)} -** ***Δt**.*

Subsequent to the operations of the clock-rate and time-lag processors, **103** and **104,** the signal synchronizer **105** implements the operation **220C** of method **200** in order to determine the phases **Δφ_{RS}** of the received location signals **RS** relative to their respective transmissions **TR₁** to **TRₖ** by the transmitter receiver utility **130** so that the position(s) of the medical modules that are connected to the signal processor **102** can be inferred based on these phase **Δφ_{RS}**. The signal synchronizer **105** processes the received location signals **RS** based on the second clock **CLK2,** the clock rate-ratio **RT** that was determined by the clock rate processor **103** and the time-lag **ΔT** that was determined by the time lag processor **104,** to determine the phases **Δφ_{RS}** of the received signals **RS**.

For instance, in some embodiments the signal synchronizer **105** digitizes the received location signals **RS** utilizing the second clock **CLK2** and utilizes the clock rate-ratio **RT** and the time-lag **ΔT** to compensate for the difference in clock rates **RT** and for the time-lag **ΔT** between the clocks **CLK1** and **CLK2.** Accordingly in such embodiments, the signal synchronizer **105** yields respective digitized received signals whose frequencies and phases adjusted relative to the first signal clock **CLK1** of the transmitter receiver utility **130.** Then, once the frequencies and phases of the received signals (or digitized versions thereof) are adjusted relative to the first signal clock **CLK1,** the signal processor **102,** or the signal synchronizer **105** may process the signals (e.g., utilizing tone detection (e.g., FFT) or clock derivation, as described above, to determine their phases **{Δφ}_{RS}** relative to the respective transmission signals that are transmitted by the transmitter receiver utility **130.**

To achieve that, in some implementations the signal synchronizer **105** is adapted to compensate for the difference in clock rates by adjusting the rate of the second clock **CLK2** in order to reduce or eliminate the difference between its rate (the second rate) and the rate of the first clock **CLK1** of the transmitter receiver utility **130.** The second clock's **CLK2** rate may for example be adjusted by a factor of the clock rate ratio **RS** such that it matches the rate of the first clock **CLK1.** Alternatively, or additionally, the signal synchronizer **105** may be adapted digitize the received signals **RS** based on the second clock **CLK2** (e.g. without rate adjustment of the clock) and then compensate for the difference **RT** in clock rates by interpolating the digitized signals based on the clock-rate-ratio **RS** (e.g. the "interpolation" here may in some cases be implemented by mere adjustment of the time labels of the signal samples by a factor of said clock-rate-ratio **RS,** which is a light weight computational process, or by interpolation of the samples themselves). Accordingly, in any of these exemplified techniques, the received signals **RS** are obtained as interpolated digital signals with their sample intervals (and hence frequencies) corresponding to the first clock rate of the first clock of the transmitter receiver utility **130.**

Moreover, the signal synchronizer **105** may be for example adapted to compensate for the time-lag **ΔT** between the first and second clocks, **CLK1** and **CLK2,** by adjusting the phase/timing of the second clock **CLK2** in order to reduce or eliminate the time-lag **ΔT.** Alternatively, or additionally, the compensation for the time lag **ΔT** may be carried out by interpolating the digitized signals to shift their samples (or sample labels) according to the time-lag **ΔT** and thereby yield the digitized signals as interpolated digital signals with samples' times shifted (and hence phases adjusted) relative to the timing of the first clock **CLK1** of the transmitter receiver utility **130**).

To this end, the phases **{Δφ}_{RS}** of the received location signals **RS** that are determined by the signal processor **102** according to the above technique relative to their respective transmissions by the transmitter receiver utility **130** are suitable for further processing to determine the positions of the position sensors **29** and/or **34,** of the medical devices **14A** and **14B** and their associated distal end portions **35A** to **35K** and **35M,** which are connected to the signal processor **102.**

In some embodiments the system **100** further implements the optional operation **230** of method **200,** and optionally includes a positioning utility **109** that is configured and operable to determining the positions of the positions sensors **29** and/or **34** of the medical devices **14A** and **14B** and their respective distal end portions **35A** to **35K** and **35M** based on the phases **{Δφ}_{RS}** inferred for their received signals **RS** by the signal processor **102.** The positioning utility **109** may be part of the signal processor **102,** or a part of a workstation **55** connected to signal processor **102** for receiving the phases **{Δφ}_{RS}**. In some embodiments the positioning utility **109** may be adapted to also process the phases **{Δφ}_{R}** of the received signals **R** that are received/sensed by position sensors of the medical devices **14C** and/or **14D**which are inferred, as indicated above, by the transmitter-receiver utility **130.** To this end the positioning utility **109** may serve for determining the positions of all the position sensors 29 and/or 34 of the medical devices, which are connected to the system, irrespectively of whether the relative phases of the location signals R/RS sensed thereby are determined by the transmitter-receiver utility **130** or by the signal processor **102** of the present invention.

As will be readily appreciated by those versed in the art the position determination itself relative the respective positions of the signal transmitters, e.g. **32,** may be implemented by various known in the art techniques (as long as the phases are correctly measured relative to the corresponding transmission signals), for example utilizing time-of-arrival (TOA), difference-time-of-arrival (DTOA), triangulation, and or any other suitable techniques or combinations thereof to determine the location(s) and/or the orientation(s) the medical module(s) relative to a reference frame (coordinates) defined by the signal transmitters e.g. **32.** The positioning utility **109** is therefore configured and operable accordingly to determine the positions of the position sensors **29** and/or **34** of with the medical devices connected to the system **10.** Accordingly, the positions of the medical devices **14A and 14B** and/or of distal end portions thereof e.g., **35A-35K** and/or **35M,** can be determined by the system **100.**

In this regard, turning back to **Figs. 1** and **2****,** as indicated above, in some implementations, a medical device **14** may be configured with a position sensor **29** arranged on a main body thereof (e.g., shaft of the catheter), while one or more additional position sensors **34** may be furnished on other distal end portions **35** of the medical device **14,** such as on flexible sections **22** of the medical device, and/or on medical instruments **26** located thereon. In some implementations one or more of the position sensors **29** and /or **34** may be implemented as single-axis sensors (SAS) and may be utilized by the system to determine the orientation of the distal end portion at which they are furnished. In some implementations one or more of the position sensors **29** and /or **34** may be implemented as three-axes sensors (TAS) which can be used for measuring both the location and orientation of the distal end portion at which they are furnished. Accordingly, as indicated above the phrase position as used herein should be understood to refer to any of a location, orientation or both of a distal end portion **35** of a medical device **14.**

To this end, it should be noted that in some implementations a medical device connected to the system may include at least one position sensor three-axes sensors (TAS) such as **29** and one or more single-axis position sensors (SAS) such as **34,** arranged at distal end portions thereof. In this case the locations of one or more of the single-axis position sensors (SAS) **34** on the distal end portions may be determined by the positioning utility based on the inferred location and orientation of the three-axes sensors (TAS) e.g., **29,** the inferred orientations of the one or more of the single-axis position sensors (SAS) **34,** and a priory data indicative of the configurations/shape that the medical device may acquire/conform to during the medical procedure.

### Examples

Example 1. A method **200** to determine positions of one or more portions **35** of a catheter's **14** distal end **28,** the method includes:
by a position sensor **29** and/or **34** at at least a portion **35** of the catheter's distal end **28,** receiving a plurality of location signals **RS** being wireless electromagnetic signals **TR₁** to **TRₖ** that were transmitted by a transmitter-receiver utility **130** with respective plurality of transmission frequencies **F_{T(1)}** to **F_{T(k)}** relative to a first clock-rate of a first clock **CLK1** associated with the transmitter-receiver utility **130;** and
processing **(220)** the location signals **RS** by a signal processor **102** associated with a second signal clock **CLK2** not synchronized with the first clock **CLK1** and having a second clock-rate, to determine phases **{Δφ}_{RS}** of the location signals **RS** relative to their respective transmissions **TR₁** to **TRₖ** by the transmitter-receiver utility **130,** the processing includes:
   A. determining **(220A)** a clock-rate-ratio **RT** as a ratio between the rates of the first and second clocks, **CLK1** and **CLK2;**
   B. determining **(220B)** a time-lag **ΔT** between the first and second signal clocks, **CLK1** and **CLK2;** and
   C. digitizing the location signals **RS** based on the second clock **CLK2**to yield respective digitized signals. The digitizing includes **(220C)** compensating for the clock-rate-ratio **RT** and compensating for the time-lag **ΔT,** such that frequencies and phases of the digitized signals are adjusted relative to the first signal clock **CLK1** of the transmitter receiver utility **130.**

Accordingly determining the phases **{Δφ}_{RS}** of the location signals **RS** relative to their respective transmissions **TR₁** to **TRₖ** by the transmitter receiver utility **130** and enabling to determine the position of the at least a portion **35** of a catheter's **14** distal end **28** based on the determined phases **{Δφ}_{RS}**.

Example 2. The method according to example 1, wherein the determining of the clock-rate-ratio between the first and second clock-rates, includes the following:
(a) providing data indicative of transmission frequency of at least one location signal of the location signals relative to the first clock-rate of the transmitter-receiver utility (based on which it is transmitted);
(b) processing the at least one location signal by the signal processor and determining the reception frequency thereof relative to the second clock-rate; and
(c) determining the clock-rate-ratio based on the transmission and reception frequencies.

Example 3. The method according to example 2, wherein the determining of the reception frequency of the at least one location signal relative to the second clock-rate includes digitizing the location signal based on the second clock to yield a digitized signal and applying tone detection processing, such as FFT, to the digitized signal.

Example 4. The method according to example 2 or 3, wherein the determining of the reception frequency of the at least one location signal relative to the second clock-rate includes applying Clock-Derivation processing to the at least one location signal.

Example 5. The method according to example 4, wherein the Clock-Derivation processing includes determining a tick-count of the second signal clock between zero-crossings of the at least one location signal and thereby determining the reception frequency thereof relative to the second signal clock.

Example 6. The method according to any of examples 1 to 5, wherein the determining of the time-lag between the first and second signal clocks, includes the following:
(a) utilizing the clock-rate-ratio for generating, by the signal processor, a dummy signal with frequency that matches a certain transmission frequency of the transmission frequencies of the location signals, and feeding the dummy signal to a receiver input of the transmitter-receiver utility, to thereby cause the transmitter-receiver utility to determine a phase difference between the dummy signal and one of the wireless electromagnetic signals that is transmitted thereby with that certain transmission frequency; and
(b) determining the time-lag between the clocks based on the phase difference and the certain transmission frequency.

Example 7. The method according to example 6, wherein the feeding of the dummy signal is carried out utilizing a channel of substantially fixed latency, and wherein the fixed latency is accounted for in the determining of the time-lag.

Example 8. The method according to example 6 or 7, wherein the generating of the dummy signal includes compensating for the clock-rate-ratio to thereby obtain the dummy signal with frequency matching the certain transmission frequency.

Example 9. The method according to any one of examples 6 to 8, including providing the phase difference between the dummy signal and the one of the wireless electromagnetic signals to the signal processor for the determining of the time-lag.

Example 10. The method according to any one of examples 1 to 9, wherein the compensating for the clock-rate-ratio includes adjusting the second clock-rate of the second signal clock in order to reduce or eliminate a difference between the first and second clock rates.

Example 11. The method according to any one of examples 1 to 10, wherein the compensating for the time lag includes adjusting the phase of the second signal clock in order to reduce or eliminate the time lag.

Example 12. The method according to any one of examples 1 to 11, wherein the compensating for the clock-rate-ratio includes re-interpolating the digitized signals based on the clock-rate-ratio to yield the digitized signals with samples corresponding to a sampling rate of the first signal clock.

Example 13. The method according to any one of examples 1 to 12, wherein the compensating for the time lag includes re-interpolating the digitized signals to shift their phases according to the time lag and thereby yield the digitized signals with phases adjusted relative to their respective transmissions by the transmitter receiver utility.

Example 14. The method according to any one of examples 1 to 13, further include determining the position of the at least portion of the catheter's distal end based on the phases of the received location signals, whereby the position is indicative of at least one of a location and orientation of the at least portion of the catheter's distal end relative to one or more reference-frame coordinates.

Example 15. A system **100** to determine a position of one or more portions **35** of a catheter's **14** distal end **28,** the system includes:
a signal processor **102** configured and operable for connecting to a position sensor **29** and/or **34** arranged at an at least a portion **35** of a catheter's **14** distal end **28,** for receiving, from the position sensor **29** and/or **34,** a plurality of location signals **RS** indicative of electromagnetic signals **TR₁** to **TRₖ** transmitted by a transmitter-receiver utility **130** with respective plurality of transmission frequencies **F_{T(1)}** to **F_{T(k)}** relative to a first clock-rate of a first clock **CLK1** of the transmitter-receiver utility **130,** and received by the position sensor **29** and/or **34;**

The signal processor **102** includes a second signal clock **CLK2** having a second clock-rate not synchronized with the first clock **CLK1.** The signal processor **102** is configured and operable to process the location signals **RS** to determine their phases **{Δφ}_{RS}** relative to their respective transmissions **TR₁** to **TRₖ** by the transmitter-receiver utility **130.** The signal processor **102** includes:
A. a clock rate processor **103** adapted to determine a clock-rate-ratio **RT** between the clock-rates of the first and second clocks, **CLK1** and **CLK2**;
B. a time lag processor **104** adapted to determine a time-lag **ΔT** between the first and second clocks, **CLK1** and **CLK2;** and
C. a signal synchronizer **105** configured and operable for utilizing the second clock **CLK2,** the clock-rate-ratio **RT** and the time-lag **ΔT,** to digitize the location signals **RS** with compensation for the clock-rate-ratio **RT** and with compensation for the time-lag **ΔT,** to thereby yield respective digitized signals with frequencies and phases adjusted relative to the first signal clock **CLK1** of the transmitter receiver utility **130.**

The signal processor thereby determines the phases **{Δφ}_{RS}** of the location signals **RS** relative to their respective transmissions **TR₁** to **TRₖ** by the transmitter receiver utility **130** and enables to determine the position of the at least portion **35** of the catheter's **14** distal end **28** based on the determined phases **{Δφ}_{RS}**.

Example 16. The system according to example 15, wherein the clock rate processor is configured and operable to determine the clock-rate-ratio by carrying out the following:
(a) obtaining data indicative of a transmission frequency of at least one location signal of the location signals relative to the first clock-rate of the transmitter-receiver utility;
(b) processing the at least one location signal to determine a reception frequency thereof relative to the second clock-rate; and
(c) determining the clock-rate-ratio based on the transmission and reception frequencies of the at least one location signal.

Example 17. The system according to example 16, wherein the clock rate processor is adapted to determine the reception frequency of the at least one location signal relative to the second clock-rate by digitizing the location signal based on the clock rate of the second clock to yield a digitized signal, and apply tone detection processing to the digitized signal to thereby determine the frequency of the location signal.

Example 18. The system according to example 16 or 17, wherein the clock rate processor includes a Clock-Derivation processor that is adapted to determine the reception frequency of the at least one location signal relative to the second clock-rate. The Clock-Derivation processor is configured and operable to determine a tick-count of the second signal clock between zero-crossings of the at least one location signal and to thereby determine the receipt frequency of the at least one location signal relative to the second signal clock.

Example 19. The system according to any one of examples 15 to 18, wherein the time lag processor is configured and operable to determine the time-lag by carrying out the following:
(a) obtaining the clock-rate-ratio from the clock rate processor and utilizing the clock-rate-ratio to generate a dummy signal with frequency matching a certain transmission frequency of the transmission frequencies;
(b) feeding the dummy signal to a receiver input of the transmitter-receiver utility, to thereby cause the transmitter-receiver utility to determine a phase difference between the dummy signal and one of the wireless electromagnetic signals which is transmitted thereby with that certain transmission frequency; and
(c) obtaining that phase difference and determining the time-lag based on that phase difference and the certain transmission frequency.

Example 20. The system according to example 19, may include a signal channel of substantially fixed and predetermined latency interconnected directly or indirectly between the signal processor and the transmitter receiver utility. The time lag processor may be adapted to feed the dummy CW signal to the transmitter receiver utility via the channel of the substantially fixed latency, and to account for the fixed latency of the channel in determination of the time-lag.

Example 21. The system according to example 19 or 20, wherein the time lag processor is adapted to carry out the compensation for the clock-rate-ratio when generating the dummy signal, such that the dummy signal is generated with frequency that matches the certain transmission frequency.

Example 22. The system according to any one of examples 19 to 21, includes a direct or indirect connection with the transmitter receiver utility. The time lag processor is adapted utilize the connection to obtain the phase difference between the dummy signal and the one of the wireless electromagnetic signals that is transmitted by the transmitter receiver utility with similar frequency as that of the dummy signal, and thereby determine said time-lag.

Example 23. The system according to any one of examples 15 to 22, wherein the signal synchronizer is configured and operable to carry out the compensation for the clock-rate-ratio by adjusting the second clock-rate of the second signal clock in order to reduce or eliminate a difference between the second clock-rate and the first clock-rate of the first clock of the transmitter receiver utility.

Example 24. The system according to any one of examples 15 to 23, wherein the signal synchronizer is configured and operable to carry out the compensation for the clock-rate-ratio by interpolating the digitized signals according to the clock-rate-ratio, to yield the digitized signals as interpolated digital signals with samples corresponding to the first clock rate of the first clock of the transmitter receiver utility.

Example 25. The system according to any one of examples 15 to 24, wherein the signal synchronizer is configured and operable to carry out the compensation for the time lag by adjusting the phase of the second clock in order to reduce or eliminate the time lag between the first and second clock.

Example 26. The system according to any one of examples 15 to 25, wherein the signal synchronizer is configured and operable to carry out the compensation for the time lag by interpolating the digitized signals to shift their phases according to the time lag and thereby yield the digitized signals as interpolated digital signals with phases adjusted relative to the timing of the first clock of the transmitter receiver utility.

Example 27. The system according to any one of examples 15 to 26, further includes a position determination utility that is configured and operable for determining the position of the at least portion of the catheter's distal end based on the phases; and wherein the position includes at least one of a location and orientation of the at least portion of the catheter's distal end, relative to one or more reference-frame coordinates.

It should also be appreciated that the examples described above are cited by way of example, and that the present disclosure is not limited to what has been particularly shown and described hereinabove. Rather, the scope of the present disclosure includes both combinations and sub-combinations of the various features described hereinabove, as well as variations and modifications thereof, which would occur to persons of ordinary skills in the art upon reading the description of the present invention and which are not disclosed in the prior art.

## Claims

1. A system (100) to determine a position of at least a portion (35) of a catheter's (14) distal end (28), the system comprises:
a signal processor (102) configured and operable for connecting to a position (29, 34) sensor arranged at an at least a portion (35) of a catheter's (14) distal end (28), for receiving, from the position sensor (29, 34), a plurality of location signals indicative of electromagnetic signals transmitted by a transmitter-receiver (130) utility with respective plurality of transmission frequencies relative to a first clock-rate of a first clock of the transmitter-receiver utility (130) and received by the position sensor (29, 34);
wherein said signal processor (102) comprises a second signal clock having a second clock-rate not synchronized with said first clock; and
wherein said signal processor (102) is configured and operable to process the location signals to determine their phases relative to their respective transmissions by the transmitter-receiver utility (130); the signal processor (102) comprising:
A. a clock rate processor (103) adapted to determine a clock-rate-ratio between said first and second clock-rates;
B. a time lag processor (104) adapted to determine a time-lag between said first and second signal clocks; and
C. a signal synchronizer (105) configured and operably for utilizing said second clock, said clock-rate-ratio and said time-lag to digitize the location signals with compensation for said clock-rate-ratio and compensation for said time-lag, to yield respective digitized signals with frequencies and phases adjusted relative to said first signal clock of the transmitter receiver utility (130);
said signal processor (102) thereby determines the phases of the location signals relative to their respective transmissions by the transmitter receiver utility (130) and thereby enables to determine the position of said at least portion (35) of the catheter's (14) distal end (28) based on said phases.

2. The system according to claim 1 wherein the clock rate processor (103) is configured and operable to determine said clock-rate-ratio by carrying out the following:
(a) obtaining data indicative of a transmission frequency of at least one location signal of said location signals relative to the first clock-rate of the transmitter-receiver utility (130);
(b) processing said at least one location signal to determine a reception frequency thereof relative to the second clock-rate; and
(c) determining said clock-rate-ratio based on said transmission and reception frequencies of the at least one location signal.

3. The system according to claim 2 wherein the clock rate processor (103) is adapted to determine the reception frequency of the at least one location signal relative to the second clock-rate by digitizing the location signal based on the clock rate of the second clock to yield a digitized signal and apply tone detection processing to the digitized signal to thereby determine the frequency of the location signal.

4. The system according to claim 2 or 3 wherein the clock rate processor (103) comprises a Clock-Derivation processor that is adapted to determine the reception frequency of the at least one location signal relative to the second clock-rate; and wherein the Clock-Derivation processor is configured and operable to determine a tick-count of the second signal clock between zero-crossings of the at least one location signal and thereby determine the receipt frequency thereof relative to the second signal clock.

5. The system according to any one of claims 1 to 4 wherein the time lag processor (104) is configured and operable to determine said time-lag by carrying out the following:
(a) obtaining said clock-rate-ratio from the clock rate processor (103) and utilizing the clock-rate-ratio to generate a dummy signal with frequency matching a certain transmission frequency of said transmission frequencies, and feed said dummy signal to a receiver input of said transmitter-receiver utility (130), to thereby cause the transmitter-receiver utility (130) to determine a phase difference between said dummy signal and one of said wireless electromagnetic signals which is transmitted thereby with said transmission frequency; and
(b) obtaining said phase difference and determining said time-lag based on said phase difference and said certain transmission frequency.

6. The system according to claim 5, comprising a signal channel (107) of substantially fixed and predetermined latency interconnected directly or indirectly between said signal processor (102) and said transmitter receiver utility (103), and wherein said time lag processor (104) is adapted to feed said dummy CW signal to said transmitter receiver utility (130) via said channel (107) of the substantially fixed latency, and to account for the fixed latency of said channel in determination of said time-lag.

7. The system according to claim 5 or 6, wherein said time lag processor (104) is adapted to carry out said compensation for the clock-rate-ratio when generating said dummy signal such that said dummy signal is generated with frequency that matches said certain transmission frequency.

8. The system according to any one of claims 5 to 7, comprising a direct or indirect connection with said transmitter receiver utility (130) and wherein said time lag processor (104) is adapted utilize said connection to obtain the phase difference between said dummy signal and said one of said wireless electromagnetic signals that is transmitted by the transmitter receiver utility (130) with similar frequency as that of the dummy signal, and thereby enable determination of said time-lag.

9. The system according to any one of claims 1 to 8, wherein the signal synchronizer (105) is configured and operable to carry out said compensation for the clock-rate-ratio by adjusting the second clock-rate of said second signal clock in order to reduce or eliminate a difference between the second clock-rate and the first clock-rates of the first clock of the transmitter receiver utility (130).

10. The system according to any one of claims 1 to 9, wherein the signal synchronizer is configured and operable to carry out said compensation for the clock-rate-ratio by interpolating the digitized signals according to said clock-rate-ratio, to yield said digitized signals as interpolated digital signals with samples corresponding to the first clock rate of the first clock of the transmitter receiver utility (130).

11. The system according to any one of claims 1 to 10, wherein the signal synchronizer is configured and operable to carry out said compensation for the time lag by adjusting the phase of said second clock in order to reduce or eliminate said time lag between the first and second clock.

12. The system according to any one of claims 1 to 11, wherein the signal synchronizer is configured and operable to carry out said compensation for the time lag by interpolating the digitized signals to shift their phases according to said time lag and thereby yield said digitized signals as interpolated digital signals with phases adjusted relative to the timing of said first clock of the transmitter receiver utility (130).

13. The system according to any one of claims 1 to 12, further comprising a position determination utility (109) that is configured and operable for determining the position of said at least portion (35) of the catheter's (14) distal end (28) based on said phases; and wherein said position comprises at least one of a location and orientation of the at least portion (35) of the catheter's distal end (28), relative to one or more reference-frame coordinates.
